(19) Europäisches Patentamt
European Patent Office
Office européen des brevets

(11) Numéro de publication : **0 025 381**
**B1**

(12) **FASCICULE DE BREVET EUROPÉEN**

(45) Date de publication du fascicule du brevet : **19.05.82**

(21) Numéro de dépôt : **80401216.9**

(22) Date de dépôt : **26.08.80**

(51) Int. Cl.³ : **C 07 C179/10**, C 07 C179/12, C 07 C178/00// C07D303/04, C07D301/14, C07D303/08

(54) **Procédé de fabrication d'acides percarboxyliques.**

(30) Priorité : **07.09.79 FR 7922398**

(43) Date de publication de la demande : **18.03.81 (Bulletin 81/11)**

(45) Mention de la délivrance du brevet : **19.05.82 Bulletin 82/20**

(84) Etats contractants désignés : **BE CH DE FR GB IT LI LU NL SE**

(56) Documents cités :
**EP - A - 0 004 407**
**BE - A - 559 665**
**FR - A - 1 492 059**
**FR - A - 2 309 552**

(73) Titulaire : **P C U K PRODUITS CHIMIQUES UGINE KUHLMANN**
**Service Propriété Industrielle Tour Manhattan**
**F-92087 Paris La Défense 2 Cedex 21 (FR)**

(72) Inventeur : **Schirmann, Jean-Pierre**
**49 Chemin de la Glacière**
**F-69600 Oullins (FR)**

**EP 0 025 381 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

# Procédé de fabrication d'acides percarboxyliques

La présente invention concerne un procédé de préparation d'acides percarboxyliques à partir d'acides carboxyliques aliphatiques miscibles à l'eau et de peroxyde d'hydrogène consistant à effectuer la réaction en présence d'une quantité catalytique d'acide ortho- ou métaborique et d'un solvant entraîneur azéotropique permettant d'éliminer continuellement l'eau du milieu réactionnel par distillation.

On sait depuis longtemps (Ber. 45, 1845, 1912) que le peroxyde d'hydrogène réagit avec les acides carboxyliques aliphatiques pour former des acides percarboxyliques selon la réaction équilibrée suivante :

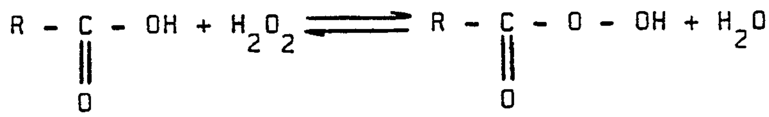 (I)

Il est connu aussi (D. Swern, Organic Peroxides, Wiley Interscience, 1970, vol. I) que si quelques rares peracides, particulièrement l'acide performique, peuvent se préparer en l'absence de catalyseur, il est généralement nécessaire pour la majorité d'entre eux de mettre en œuvre un catalyseur pour diminuer la durée de la réaction.

En effet, étant donné l'instabilité des acides percarboxyliques, cette réaction est généralement conduite à température modérée. Dans ces conditions l'état d'équilibre n'est atteint qu'après plusieurs heures de réaction et une telle durée n'est pas admissible dans le cadre d'un procédé industriel. Il est donc nécessaire d'avoir recours à l'emploi d'un catalyseur.

Les catalyseurs qui ont été proposés et utilisés jusqu'à maintenant sont les acides minéraux ou organiques forts, comme l'acide phosphorique, l'acide sulfurique, l'acide chlorhydrique, les acides alkyl- ou arylsulfoniques, comme l'acide méthanesulfonique, l'acide paratoluènesulfonique, l'acide trifluoracétique, ainsi que les résines cationiques acides telles que les résines commerciales « Dovex 50 » ou « Amberlite IR-120 ».

Ce processus catalytique a fait l'objet de nombreuses études (D. Swern, « Organic Peroxides », Wiley Interscience 1970, vol. I, pages 313-369 et pages 428-439). Il a été montré que la première étape de la réaction correspond à la protonation de la fonction acide carboxylique entraînant la formation d'une structure oxonium susceptible de réagir avec $H_2O_2$ pour conduire à l'acide percarboxylique selon le schéma :

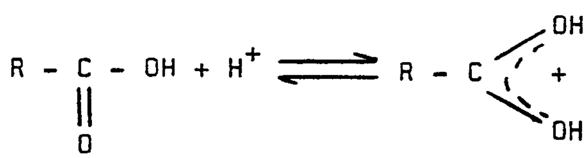 (II)

$$H_2O_2 \rightleftharpoons HOO^- + H^+ \quad \text{(III)}$$

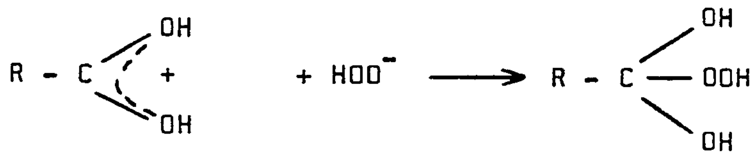 (IV)

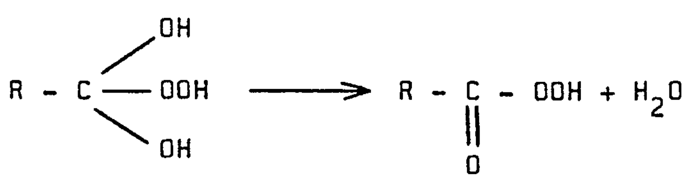 (V)

Le peroxyde d'hydrogène est le plus souvent mis en œuvre sous forme de solutions aqueuses

commerciales contenant de 30 à 70 % d'eau. Comme la réaction fournit aussi une molécule d'eau par molécule d'acide percarboxylique, il est clair que l'état d'équilibre (1) est atteint bien avant que le peroxyde d'hydrogène soit totalement transformé. Dans ces conditions le produit de la réaction est en fait un mélange d'acide carboxylique, de peroxyde d'hydrogène, d'acide percarboxylique, d'eau et de catalyseur acide fort.

De nombreux moyens ont été proposés pour déplacer l'équilibre vers le droite, en vue d'utiliser le plus complètement possible le peroxyde d'hydrogène ou obtenir un peracide exempt de l'un ou l'autre des autres constituants de l'équilibre, souvent gênants pour l'utilisation envisagée. En effet, s'il est parfois possible d'utiliser tel quel le mélange et d'achever la transformation du peroxyde d'hydrogène in situ, lors de certaines époxydations par exemple, ce processus simple est souvent inadéquat en raison des réactions secondaires que peuvent provoquer les autres constituants du mélange : oxydation par le peroxyde d'hydrogène, hydrolyse par l'eau, action néfaste du catalyseur acide fort.

La plupart des techniques décrites font appel à des moyens connus de déshydratation de milieux organiques. On a proposé ainsi d'effectuer la réaction en présence d'une quantité d'agents déshydratants suffisante pour se combiner à l'eau présente ou formée au cours de la réaction. Mais les agents préconisés jusqu'à présent ne donnent pas entière satisfaction et de plus engendrent des problèmes de séparation difficiles à résoudre sur le plan pratique. C'est ainsi que la demanderesse, dans son brevet français 1 492 059, avait proposé d'utiliser l'acide métaborique comme agent de fixation stoechiométrique de l'eau, en présence de catalyseurs acides forts. Cette technique permet effectivement d'atteindre de très hauts taux de transformation du peroxyde d'hydrogène, mais nécessite la séparation de l'acide orthoborique résultant de l'hydratation de l'acide métaborique et qui est insoluble dans le milieu. La nécessité de manipuler un solide éventuellement imprégné de peracide puis de lui faire subir une déshydratation à haute température, rend ce procédé délicat et difficile à mettre en œuvre.

On a aussi proposé comme dans les brevets des Etats-Unis d'Amérique n° 2 877 266 et 2 814 641 de n'opérer qu'avec un très léger excès d'acide carboxylique, mais en présence d'un catalyseur acide minéral fort et d'un entraîneur azéotropique pour éliminer l'eau et déplacer l'équilibre (1) vers la droite. Une telle pratique est effectivement excellente quant au rendement en acide percarboxylique par rapport au peroxyde d'hydrogène engagé.

Cependant toutes ces techniques présentent en commun un défaut majeur : le catalyseur acide fort nécessaire pour accélérer le processus réactionnel, engendre le plus souvent lors de l'utilisation des solutions de peracides, des réactions parasites entraînant des baisses de rendements importantes. Il est bien connu par exemple que dans les réactions d'époxydation des oléfines par les peracides, l'époxyde formé est facilement ouvert et transformé en mélange de mono et diester sous l'effet des catalyseurs acides forts.

Il est vrai que l'acide fort peut être avantageusement neutralisé, mais alors le sel correspondant est en général insoluble dans le milieu et cela pose des problèmes de séparation non négligeables sur le plan pratique. Parfois même ce sel est aussi bon catalyseur de réactions parasites que l'acide lui-même.

C'est pourquoi on a proposé récemment, comme dans les brevets français n° 2 359 132 et n° 2 300 085 une méthode de fabrication de solutions organiques d'acides percarboxyliques en deux étapes, qui consiste à faire réagir une solution aqueuse contenant de 10 à 45 % d'acide sulfurique servant de déshydratant et de catalyseur et de 20 à 35 % de peroxyde d'hydrogène avec de l'acide propionique, puis à extraire l'acide perpropionique formé à l'aide d'un solvant tel que le benzène ou le dichloropropane. La phase aqueuse doit être concentrée pour éliminer l'eau apportée par le peroxyde d'hydrogène et celle formée au cours de la réaction. La phase organique est lavée à l'eau pour éliminer $H_2SO_4$, puis séchée par distillation azéotropique. Cette solution permet effectivement d'obtenir avec un bon rendement sur le peroxyde d'hydrogène une solution organique d'acide perpropionique anhydre débarrassée de catalyseur acide fort. Cependant il s'agit là d'une technique très lourde à mettre en œuvre et par conséquent très coûteuse.

Poursuivant ses travaux dans le domaine de l'utilisation du peroxyde d'hydrogène en chimie organique, la demanderesse vient de découvrir qu'il est possible de parvenir au même résultat, c'est-à-dire obtenir une solution organique anhydre d'acide percarboxylique exempte de toute trace d'acide minéral fort, en faisant réagir l'acide carboxylique et le peroxyde d'hydrogène en présence de quantités catalytiques d'acide orthoborique ou d'acide métaborique et d'un solvant entraîneur azéotropique permettant d'éliminer continuellement du milieu réactionnel l'eau apportée par la solution aqueuse de peroxyde d'hydrogène ainsi que l'eau résultant de la réaction.

Les acides orthoborique $B(OH)_3$ et métaborique $(HOBO)_n$ sont bien connus pour être des acides minéraux faibles dont l'action, en tant qu'acide, ne peut être comparée aux acides forts tels que $H_2SO_4$. Il s'agit donc sans aucun doute d'un processus catalytique différent, dont la nature n'a cependant pas pu encore être élucidée.

Les acides carboxyliques concernés par l'invention sont les acides carboxyliques aliphatiques solubles dans l'eau, c'est-à-dire les acides formique, acétique, propionique, butyriques.

L'entraîneur azéotropique peut être choisi de façon avantageuse parmi les solvants de point d'ébullition inférieur à 100° et formant un hétéroazéotrope avec l'eau. On peut citer à titre d'exemples non limitatifs des solvants chlorés tels que le chloroforme, le tétrachlorure de carbone, le chlorure de méthylène, le dichloro-1,2 éthane, le dichloropropane, des solvants hydrocarbonés tels que le cyclohexane, le benzène, le toluène, des esters tels que les formiates, acétates, propionates, butyrates, isobutyrates de méthyle, d'éthyle, de propyle, d'isopropyle, de n-butyle.

Le peroxyde d'hydrogène peut être mis en œuvre dans le cadre de l'invention aussi bien sous forme anhydre, que sous forme de solution aqueuse commerciale titrant de 30 à 70 % en poids.

Le procédé conforme à l'invention consiste donc à mettre en contact l'acide carboxylique, l'entraîneur azéotropique, le catalyseur et le peroxyde d'hydrogène en éliminant continuellement l'eau du milieu réactionnel par distillation azéotropique.

La température à laquelle est réalisée la réaction est comprise entre 40° et 100° et préférentiellement entre 40° et 70°. Selon la température choisie et le système réactionnel mis en œuvre, l'élimination pourra se faire en opérant à pression atmosphérique ou sous pression réduite. La pression peut donc varier entre 20 mm de mercure et 760 mm de mercure.

La durée de la réaction dépend de la nature du catalyseur, de la nature de l'acide carboxylique, de la nature de l'entraîneur azéotropique et de la température choisie. Elle peut aller de quelques minutes à plusieurs heures. Les réactifs peuvent être engagés en quantités équimoléculaires, mais on peut aussi utiliser un excès ou un défaut molaire de l'un ou l'autre des réactifs. A titre indicatif on peut engager de 0,1 à 10 moles d'acide carboxylique par mole de peroxyde d'hydrogène, mais on engage préférentiellement de 1 à 5 moles.

Le catalyseur est utilisé à raison de 0,001 à 0,1 mole d'acide borique par mole de peroxyde d'hydrogène. On préfère cependant un rapport molaire compris entre 0,001 et 0,01 mole par mole de peroxyde d'hydrogène engagé.

La quantité de solvant entraîneur azéotropique est comprise entre 50 et 75 % en poids du mélange réactionnel, de telle sorte que l'on puisse régler à volonté le point d'ébullition du mélange et éliminer de façon efficace l'eau.

Les réactifs peuvent être utilisés sous leur forme commerciale habituelle. Le peroxyde d'hydrogène en particulier peut être mis en œuvre sous forme de solutions aqueuses commerciales titrant de 30 à 70 % en poids. Il peut être avantageux d'ajouter au mélange réactionnel des produits stabilisants du peroxyde d'hydrogène, tels que phosphates, polyphosphates et dérivés de l'acide éthylènediaminetétracétique.

La solution d'acide percarboxylique ainsi obtenue peut alors servir à réaliser l'oxydation de très nombreux composés organiques tels que oléfines, cétones, amines, composés aromatiques et dérivés soufrés au cours d'une deuxième opération. Cependant il n'est pas toujours nécessaire d'avoir recours à cette procédure et l'on peut parfois avantageusement réaliser les deux opérations en même temps, c'est-à-dire la synthèse du peracide et sa consommation immédiate par la molécule à oxyder. Cela constitue une variante du procédé conforme à l'invention. C'est ainsi que lorsque le composé organique que l'on veut oxyder par l'acide percarboxylique, forme un hétéroazéotrope avec l'eau, il peut être utilisé comme entraîneur azéotropique et dans le même temps réagir avec l'acide percarboxylique au fur et à mesure de la formation de celui-ci. On peut citer à titre d'exemple l'époxydation du cyclohexène ou du chlorure d'allyle par l'acide peracétique ou l'acide perpropionique. Une telle procédure est particulièrement simple à mettre en œuvre et offre une grande sécurité puisqu'elle permet d'éviter toute accumulation de peracide dans le milieu réactionnel.

Dans le cadre de cette variante, si le composé à oxyder ne forme pas d'hétéroazéotrope avec l'eau, il est bien sûr tout-à-fait possible d'opérer en présence d'un solvant entraîneur azéotropique.

Les exemples suivants illustrent de façon non limitative la présente invention.

## Exemples 1 à 7

Dans un réacteur de 250 $cm^3$ équipé d'une colonne de distillation de 5 plateaux Oldershaw surmontée d'un condenseur à reflux, on place 50 g d'acide propionique, 70 g de solvant entraîneur azéotropique, 0,2 g de catalyseur, (sauf dans les exemples 1 et 2 qui sont donnés à titre comparatif) et 0,1 g de phosphate disodique. Ce mélange est porté à reflux, puis on introduit progressivement 0,1 mole de peroxyde d'hydrogène sous forme de solution aqueuse à 70 % en poids. Le condenseur est conçu de telle façon que seule la phase organique condensée est refluée dans la colonne, la phase aqueuse décantée étant soutirée de façon continue. Les conditions de réaction et les résultats sont consignés dans le tableau I.

## Exemple 8

Dans un réacteur de 500 $cm^3$ équipé d'une colonne de distillation de 10 plateaux Oldershaw surmontée d'un condenseur à reflux du même type que celui décrit ci-dessus, on place 125 g d'acide propionique, 175 g de dichloro-1,2 éthane, 0,5 g d'acide orthoborique $H_3BO_3$ et 0,1 g de phosphate disodique. On porte à reflux sous une pression de 150 mm de mercure. La température du milieu réactionnel est de 50°. On additionne progressivement 0,3 mole de peroxyde d'hydrogène sous forme de solution aqueuse à 70 % en poids. Après deux heures de réaction au cours desquelles l'eau est éliminée de façon continuelle par distillation azéotropique, on dose dans le milieu 0,24 mole d'acide perpropionique ainsi que 0,027 mole de peroxyde d'hydrogène, alors que la phase aqueuse distillée contient 0,032 mole de peroxyde d'hydrogène.

Exemple 9

Dans un réacteur tel que décrit à l'exemple 1, on place 50 g d'acide acétique, 80 g de chlorure d'allyle ainsi que 0,2 g d'acide orthoborique $H_3BO_3$. On porte à reflux et la température du milieu réactionnel se fixe à 55 °C. On ajoute en 15 minutes, 0,050 mole de peroxyde d'hydrogène sous forme de solution aqueuse à 70 % et l'on élimine en continu l'eau apportée par $H_2O_2$ et celle formée au cours de la réaction. Après deux heures de réaction on dose dans le milieu réactionnel 0,042 mole d'épichlorhydrine, 0,002 mole d'acide peracétique et 0,001 mole de peroxyde d'hydrogène alors que le distillat contient 0,004 mole de peroxyde d'hydrogène.

Exemple 10

Dans un réacteur tubulaire de 15 m de longueur et de diamètre ∅ = 2 mm, on introduit en continu après passage dans un mélangeur 100 g/h de solution d'acide perpropionique préparée selon l'exemple 8 et titrant 6,7 % de peracide et 0,15 % de peroxyde d'hydrogène, ainsi que 21 g/h de propylène. La température du réacteur est maintenue à 50 °C. La pression dans le réacteur est de 8 bars. A la sortie du réacteur, on procède en continu à la décompression du mélange réactionnel. La phase gazeuse est lavée à l'eau dans une colonne de lavage pour récupérer l'oxyde de propylène entraîné. La phase liquide est refroidie. L'analyse des produits de la réaction montre qu'il sort du réacteur 0,01 mole d'acide perpropionique et qu'il s'est formé 4 g/h d'oxyde de propylène.

(voir Tableau I, p. 6)

Tableau I

| Exemples | Acide carboxylique | Catalyseur | Solvant | T° C | Pression mm Hg | Durée mn | $H_2O_2$ restante mmoles | Peracides formés mmoles | $H_2O_2$ distillée mmoles |
|---|---|---|---|---|---|---|---|---|---|
| 1 | Propionique | - | Cyclohexane | 93° | 760 | 30 | 14 | 12 | 35 |
| 2 | " | - | Dichloréthane | 50° | 150 | 180 | 20 | 5 | 3 |
| 3 | " | $H_3BO_3$ | Cyclohexane | 94° | 760 | 30 | 2 | 50 | 20 |
| 4 | " | " | Dichloréthane | 94° | 760 | 30 | 6 | 75 | 7 |
| 5 | " | " | " | 50° | 150 | 180 | 3 | 82 | 14 |
| 6 | " | $(HBO_2)_n$ | " | 50° | 150 | 180 | 3,9 | 90 | 5 |
| 7 | Acétique | $H_3BO_3$ | " | 50° | 180 | 180 | 7 | 87 | 6 |

**Revendications**

1. Procédé de préparation d'acide percarboxylique aliphatique par action du peroxyde d'hydrogène sur un acide carboxylique aliphatique miscible à l'eau, caractérisé par le fait que l'on opère en phase liquide homogène à une température comprise entre 40 °C et 100 °C, en présence d'un acide borique comme catalyseur, utilisé à raison de 0,001 à 0,1 mole par mole de peroxyde d'hydrogène, et d'un solvant organique susceptible de former un hétéroazéotrope avec l'eau, et que l'on élimine de façon continue par distillation azéotropique l'eau introduite avec le peroxyde d'hydrogène ainsi que l'eau formée au cours de la réaction.

2. Procédé suivant la revendication 1, caractérisé par l'utilisation comme catalyseur de l'acide orthoborique $B(OH)_3$.

3. Procédé suivant la revendication 1, caractérisé par l'utilisation comme catalyseur de l'acide métaborique $(HO-B=O)_n$.

4. Procédé suivant l'une quelconque des revendications 1 à 3, où l'on utilise comme entraîneur azéotropique un solvant organique chloré.

5. Procédé suivant l'une quelconque des revendications 1 à 3, où l'on utilise comme entraîneur azéotropique le cyclohexane.

6. Procédé suivant l'une quelconque des revendications 1 à 5, où l'acide carboxylique aliphatique est de l'acide acétique.

7. Procédé suivant l'une quelconque des revendications 1 à 5, où l'acide carboxylique aliphatique est de l'acide propionique.

8. Procédé suivant l'une quelconque des revendications 1 à 7, où l'on utilise comme entraîneur azéotropique un composé organique susceptible à la fois de former un hétéroazéotrope avec l'eau et de réagir avec l'acide percarboxylique au fur et à mesure de sa formation dans le milieu réactionnel.

9. Procédé suivant la revendication 8 où l'entraîneur azéotropique est une oléfine.

10 Procédé suivant la revendication 9 où l'entraîneur azéotropique est le cyclohexène.

11. Procédé suivant la revendication 9 où l'entraîneur azéotropique est le chlorure d'allyle.

**Claims**

1. Process for the preparation of an aliphatic percarboxylic acid by reacting hydrogen peroxide with a water-miscible aliphatic carboxylic acid, characterised in that the reaction is carried out in the homogeneous liquid phase at a temperature of between 40 °C and 100 °C, in the presence of a boric acid as a catalyst, used in an amount of 0.001 to 0.1 mol per mol of hydrogen peroxide, and in the presence of an organic solvent capable of forming a heterogeneous azeotrope with water, and in that the water introduced with the hydrogen peroxide, and also the water formed during the reaction, are removed continuously by azeotropic distillation.

2. Process according to Claim 1, characterised in that orthoboric acid, $B(OH)_3$ is used as the catalyst.

3. Process according to Claim 1, characterised in that metaboric acid, $(HO-B=O)_n$, is used as the catalyst.

4. Process according to any one of Claims 1 to 3, in which a chlorinated organic solvent is used as the azeotropic entraining agent.

5. Process according to any one of Claims 1 to 3, in which cyclohexane is used as the azeotropic entraining agent.

6. Process according to any one of Claims 1 to 5, in which the aliphatic carboxylic acid is acetic acid.

7. Process according to any one of Claims 1 to 5, in which the aliphatic carboxylic is propionic acid.

8. Process according to any one of Claims 1 to 7, in which the organic compound used as the azeotropic entraining agent is capable both of forming a heterogeneous azeotrope with water and of reacting with the percaboxylic acid as it is formed in the reaction medium.

9. Process according to Claim 8, in which the azeotropic entraining agent is an olefine.

10. Process according to Claim 9, in which the azeotropic entraining agent is cyclohexene.

11. Process according to Claim 9, in which the azeotropic entraining agent is allyl chloride.

**Ansprüche**

1. Verfahren zur Herstellung einer aliphatischen Percarboxylsäure durch Reaktion von Wasserstoffperoxid mit einer in Wasser mischbaren aliphatischen Carboxylsäure, dadurch gekennzeichnet, daß man in einer homogenen, flüssigen Phase bei einer Temperatur zwischen 40 °C und 100 °C arbeitet, in Gegenwart einer Borsäure als Katalysator, die in einer Menge von 0,001 bis 0,1 Mol je Mol Wasserstoffperoxid eingesetzt wird und eines organischen Lösungsmittels, das mit Wasser ein Heteroazeotrop bilden kann und daß man durch azeotrope Destillation kontinuierlich das mit dem Wasserstoffperoxid eingeführte und das im Verlauf der Reaktion gebildete Wasser entfernt.

2. Verfahren nach Anspruch 1, gekennzeichnet durch die Verwendung von Orthoborsäure $B(OH)_3$ als Katalysator.

3. Verfahren nach Anspruch 1, gekennzeichnet durch die Verwendung von Methaborsäure $(HO-B=O)_n$ als Katalysator.

4. Verfahren nach einem der Ansprüche 1 bis 3, bei dem man als azeotropen Träger ein chloriertes, organisches Lösungsmittel verwendet.

5. Verfahren nach einem der Ansprüche 1 bis 3, bei dem man als azeotropen Träger Cyclohexan verwendet.

6. Verfahren nach einem der Ansprüche 1 bis 5, bei dem die aliphatische Carboxylsäure Essigsäure ist.

7. Verfahren nach einem der Ansprüche 1 bis 5, bei dem die aliphatische Carboxylsäure Propionsäure ist.

8. Verfahren nach einem der Ansprüche 1 bis 7, bei dem man als azeotropen Träger eine organische Verbindung verwendet, die gleichzeitig mit Wasser ein Heteroazeotrop bilden und mit der Carboxylsäure in dem Maße reagieren kann, wie diese im Reaktions-medium gebildet wird.

9. Verfahren nach Anspruch 8, bei dem der azeotrope Träger ein Olefin ist.

10. Verfahren nach Anspruch 9, bei dem der azeotrope Träger Cyclohexen ist.

11. Verfahren nach Anspruch 10, bei dem der azeotrope Träger Allylchlorid ist.